# EUROPEAN PATENT APPLICATION

(11) **EP 3 258 255 A1**
(43) Date of publication of application: **20.12.2017**
(21) Application number: 16182888.4
(22) Date of filing: 04.08.2016
(51) Int. Cl.: G01N 27/327, G01R 1/073, G01R 31/02, G01R 31/28, H01R 13/24

(54) **UNIVERSAL CONNECTION RECOGNITION SYSTEM**

(30) Priority: 16.06.2016 CN 201610431497
(71) Applicant: Kingpak Technology Inc., Hsin-Chu Hsien 30267 (TW)
(72) Inventor: CHAN, Chia-Hao, Hsin-Chu Hsien (TW); CHEN, Han-Hsing, Hsin-Chu Hsien (TW)
(74) Representative: Viering, Jentschura & Partner mbB Patent- und Rechtsanwälte

(57) **Abstract**

The present invention discloses a universal connection recognition system which includes a universal connector, a pin recognition module and a signal detection module, wherein the universal connector is composed of plural testing pins that are electrically connected to plural connection pads to be tested, while at least one testing pin is connected to each connection pad to be tested. With the implementation of the present invention, complex production process or equipment is not required thus enormously reduce the implementation cost; the universal connection recognition system can be applied to connections of great diversity of inspection instruments or equipment to thus make more applications possible; and with the learning capability of the universal connection recognition system, any inspection instrument or equipment once connected is memorized to have the capability of achieving automatic and exact pin compatibility when the inspection instrument or equipment is connected again.

## Description

### Field of the Invention

The present invention relates to a connection recognition system configured for detection purposes and more particularly to a universal connection recognition system with a pin recognition module and a detection module.

### Background of the Invention

Detection instruments or devices have rapidly come into extensive use in modern life, especially those related to physical health or medical treatment.

Today, detection instruments or devices are available in large numbers and a great variety, with marked differences in size, form, location, and number of their probes, pads, or contacts for detection. The differences not only complicate the collection and compilation of detection data, but also make it difficult to improve the utilization efficiency of those data effectively.

In the light of this, it has been an important issue in the detection technology industry, or even the entire medical application industry, to develop a simple and effective technique or detection system that can be implemented at low cost by dispensing with a complex manufacturing process and expensive production equipment, that features an innovative structural design compatible with different detection instruments or devices and therefore widely applicable, and that has a learning function for automatically applying the correct pin-grouping arrangement to an instrument or device that is connected to the system again, thereby satisfying the demands of electronic devices with a detection or recognition system while greatly enhancing the quality of medical examination and the health of humanity.

### Summary of the Invention

The present invention discloses a universal connection recognition system which includes a universal connector, a pin recognition module, and a detection module. The universal connector is composed of a plurality of detection pins to be electrically connected with a plurality of external terminals to be detected. More specifically, each external terminal to be detected is electrically connected with at least one of the detection pins. This universal connection recognition system can be implemented at low cost without a complicated manufacturing process or costly production equipment. Moreover, the system is compatible with various detection instruments or devices and therefore has a wide range of applications. Last but not least, the system has a learning function to enable automatic application of the correct pin-grouping arrangement to an instrument or device that is connected to the system again.

The present invention provides a universal connection recognition system which includes a universal connector, a pin recognition module, and a detection module. The universal connector is composed of a plurality of detection pins. The pin recognition module is configured to send an identifying signal to one of the detection pins, read the signal of each of the other detection pins, and then identify and group the detection pins to form a corresponding pin combination. The detection module is configured to send a test signal to the corresponding pin combination and to receive and process a feedback signal sent by the corresponding pin combination in response to the test signal. The detection pins are electrically connected with a plurality of external terminals to be detected, and each external terminal to be detected is electrically connected with at least one of the detection pins.

Implementation of the present invention at least produces the following advantageous effects:
1. A low implementation cost can be achieved because neither a complicated manufacturing process nor expensive production equipment is required.
2. A wide range of applications are made possible by the system's compatibility with various detection instruments or devices.
3. Thanks to the learning function of the system, any instrument or device that is connected to the system again will be automatically connected with the correctly grouped detection pins.

The features and advantages of the present invention are detailed hereinafter with reference to the preferred embodiments. The detailed description is intended to enable a person skilled in the art to gain insight into the technical contents disclosed herein and implement the present invention accordingly. In particular, a person skilled in the art can easily understand the objects and advantages of the present invention by referring to the disclosure of the specification, the claims, and the accompanying drawings.

### Brief Description of the Drawings

The invention as well as a preferred mode of use, further objectives and advantages thereof will be best understood by reference to the following detailed description of illustrative embodiments when read in conjunction with the accompanying drawings, wherein:
FIG. 1 is a schematic diagram of the universal connection recognition system in an embodiment of the present invention;
FIG. 2 is a schematic diagram of the universal connection recognition system in another embodiment of the present invention, wherein the system has a control unit;
FIG. 3 is a schematic diagram of the universal connection recognition system in yet another embodiment of the present invention, wherein the pin recognition module includes a digital signal generating unit and a pin recognition unit;
FIG. 4 is a schematic diagram of the universal connection recognition system in still another embodiment of the present invention, wherein the detection module includes an analog signal generating unit and an impedance detection unit;
FIG. 5 is a flowchart showing the process flow of the pin recognition module in an embodiment of the present invention;
FIG. 6 is a flowchart showing the process flow of the detection module in an embodiment of the present invention;
FIG. 7 schematically shows the detection operation of the detection module in an embodiment of the present invention;
FIG. 8A is a perspective view of the detection pins in an embodiment of the present invention, wherein the detection pins are arranged in a two-dimensional (2D) array;
FIG. 8B is a perspective view of the detection pins in another embodiment of the present invention, wherein the detection pins are arranged in a three-dimensional (3D) array; and
FIG. 9 is a perspective view showing how the detection pins in an embodiment of the present invention detect the to-be-detected terminals, wherein the detection pins are arranged in a 3D array.

### Detailed Description of the Invention

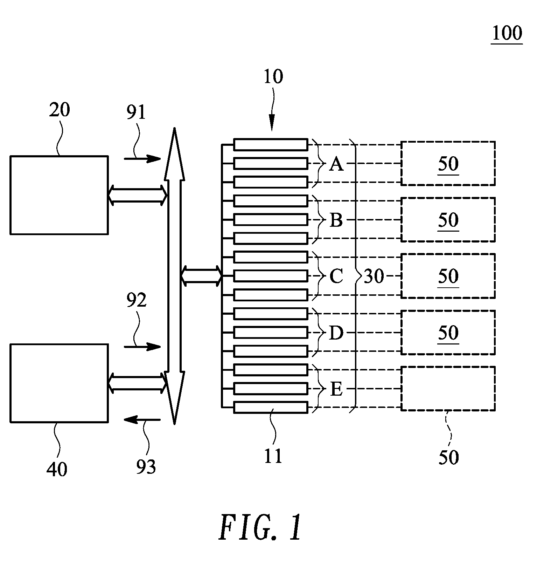

Referring to FIG. 1, the universal connection recognition system 100 in an embodiment of the present invention includes a universal connector 10, a pin recognition module 20, and a detection module 40.

As shown in FIG. 1, the universal connector 10 is composed of a plurality of detection pins 11. Each detection pin 11 can be a gold finger or a pogo pin. The number of the detection pins 11 and the shape of each detection pin 11 depend on the intended applications or production requirements; the present invention does not have special limitations in this regard.

The detection pins 11 are configured mainly for electrical connection with a plurality of external terminals 50 to be detected. More specifically, each to-be-detected terminal 50 is to be electrically connected with at least one detection pin 11. In other words, the thickness or width of each detection pin 11 can be less than that of each to-be-detected terminal 50.

The to-be-detected terminals 50 can be the terminals of a detection instrument or device of any brand or configuration. For example, the to-be-detected terminals 50 can be the connection terminals of a blood glucose meter of any brand or configuration.

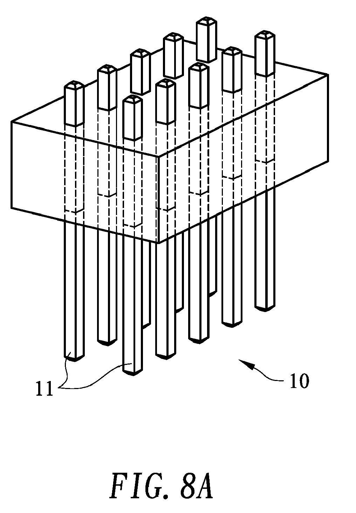

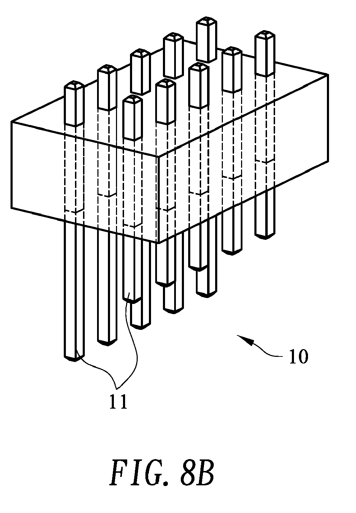

In addition, the detection pins 11 can be arranged in a two-dimensional (2D) array, as shown in FIG. 8A, or a three-dimensional (3D) array, as shown in FIG. 8B. Proper arrangement of the detection pins 11 not only facilitates signal communication, but also may increase the area of contact between the detection pins 11 and the to-be-detected terminals 50. When the to-be-detected terminals 50 are relatively thick or are arranged over a relatively large area, arranging the detection pins 11 in a 3D array helps provide error-free contact.

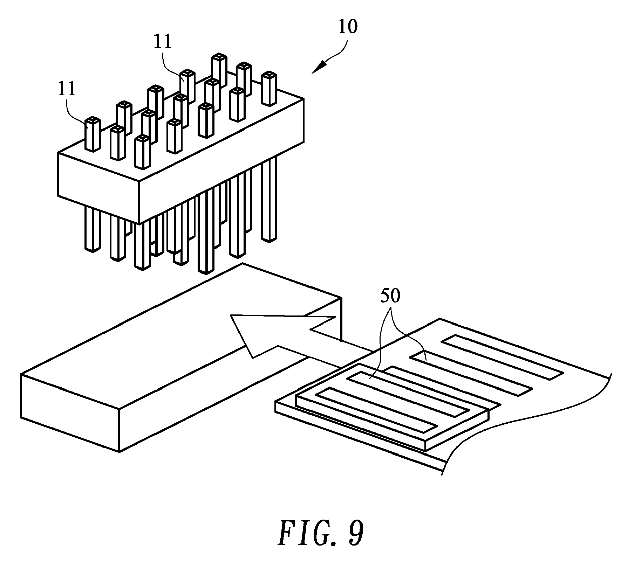

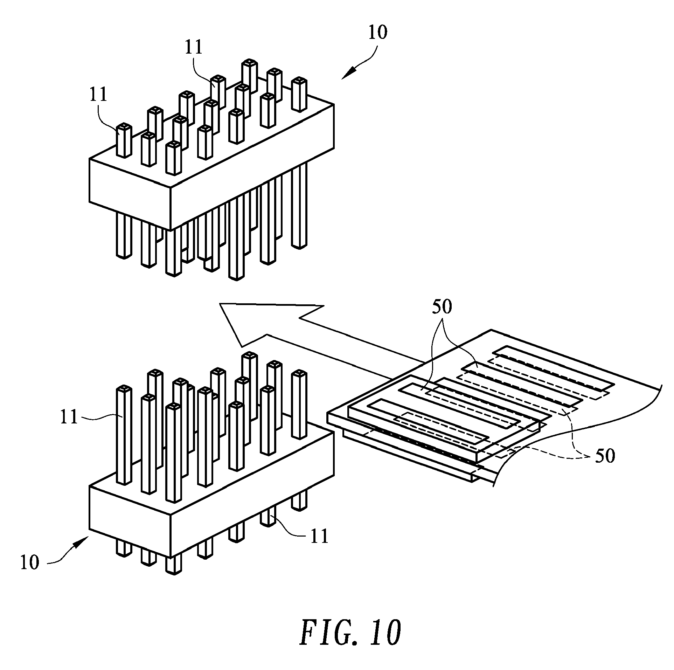

FIG. 9 shows how the detection pins 11, arranged in a 3D array, are used to detect the to-be-detected terminals 50. Whether or not the contact portions of the to-be-detected terminals 50 have a uniform thickness or height, the 3D array of detection pins 11 can detect the to-be-detected terminals 50 without error.

Referring back to FIG. 1, the pin recognition module 20 is configured to send an identifying signal 91 to one of the detection pins 11, read the signal of each of the other detection pins 11, and then identify and group the detection pins 11 to form a corresponding pin combination 30. The identifying signal 91 can be a digital signal. In one embodiment of the present invention, the identifying signal 91 is a current signal such that, when several detection pins are in contact with the same to-be-detected terminal 50 and the current signal is sent to the to-be-detected terminal 50 via a specific one of those detection pins, the rest of the detection pins in contact with the to-be-detected terminal 50 each detect a corresponding current signal and are therefore classified as in the same group as that specific detection pin. The identifying signal 91, however, is not necessarily a current signal, provided that the identifying signal 91 can be used to determine whether a plurality of detection pins are in contact with the same to-be-detected terminal.

As demonstrated by the embodiment shown in FIG. 1, some detection pins 11 are classified as group A, some detection pins 11 are classified as group B, some detection pins 11 are classified as group C, some detection pins 11 are classified as group D, and some detection pins 11 are classified as group E. Groups A through E define a corresponding pin combination 30.

When the to-be-detected terminals 50 are the connection terminals of a blood glucose meter of a certain brand and configuration, the corresponding pin combination 30 obtained by the foregoing identifying and grouping process includes a pin-grouping arrangement that matches the connection terminals perfectly.

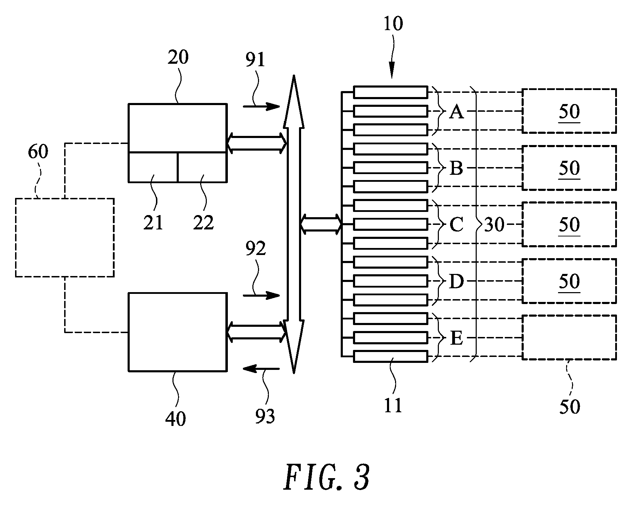

Referring to FIG. 3, the pin recognition module 20 of the universal connection recognition system 100 may further include a digital signal generating unit 21 and a pin recognition unit 22. The digital signal generating unit 21 is configured to generate and send out a digital signal while the pin recognition unit 22 is configured to identify and group the detection pins 11.

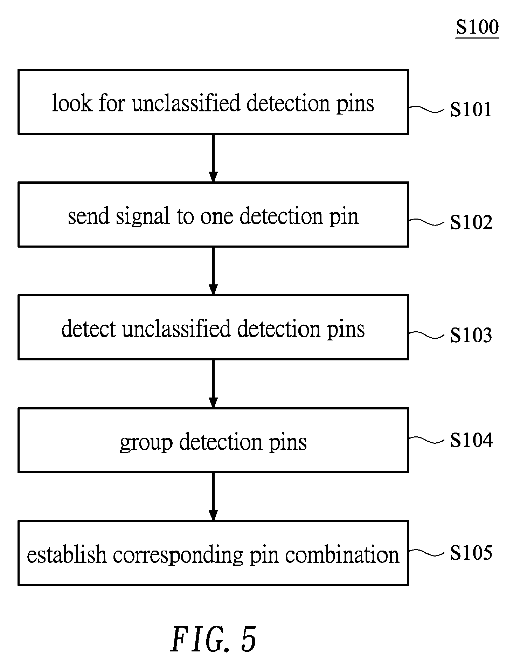

FIG. 5 shows an embodiment of the recognition process S100 performed by the pin recognition module 20. As stated above with regard to the function of the pin recognition module 20, the process S100 may include: looking for any unclassified detection pin (step S101), sending a signal to one detection pin (step S102), detecting the other detection pins (step S103), grouping the detection pins (step S104), and establishing a corresponding pin combination (step S105).

To look for any unclassified detection pin (step S101), the universal connection recognition system 100 reads pin-corresponding data to identify any unclassified detection pin 11. The pin-corresponding data can be stored in the universal connection recognition system 100 or read in from an external source.

To send a signal to one detection pin (step S102), the universal connection recognition system 100 sends an identifying signal 91 to one of the detection pins 11.

To detect the other detection pins (step S103), the universal connection recognition system 100 reads the signals sent by all the detection pins 11 except for the detection pin 11 to which the identifying signal 91 was sent.

To group the detection pins (step S104), the universal connection recognition system 100 identifies and groups the detection pins 11 according to the signals read from all the detection pins 11 except for the detection pin 11 to which the identifying signal 91 was sent.

To establish a corresponding pin combination (step S105), the universal connection recognition system 100 defines the identified and grouped detection pins 11 as a corresponding pin combination 30.

Referring again to FIG. 1, the detection module 40 is configured to send a test signal 92 to the corresponding pin combination 30 and then receive and process a feedback signal 93 sent by the corresponding pin combination 30 in response to the test signal 92.

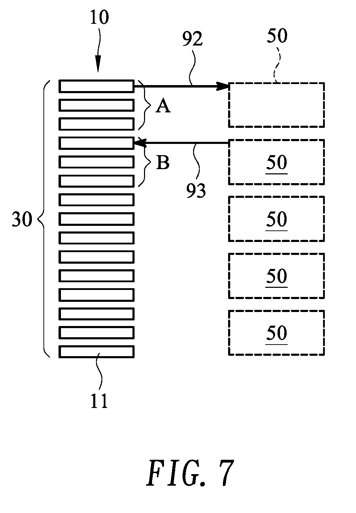

As shown in FIG. 1 and FIG. 7, the test signal 92 sent by the detection module 40 can be an analog signal such as a voltage signal or a current signal.

In FIG. 7, where the test signal 92 is a voltage signal by way of example, the test signal 92 of a certain voltage value is input to group A in the corresponding pin combination 30, and then the voltage value of the feedback signal 93 detected from a group other than group A (e.g., group B) is read. By calculating the difference between the input voltage value and the read voltage value, the impedance value between group A and group B is obtained.

By the same token, the impedance value between each two groups can be obtained, thereby acquiring the impedance characteristics of the entire corresponding pin combination 30, and hence of each two connection terminals of the corresponding blood glucose meter (or other detection device).

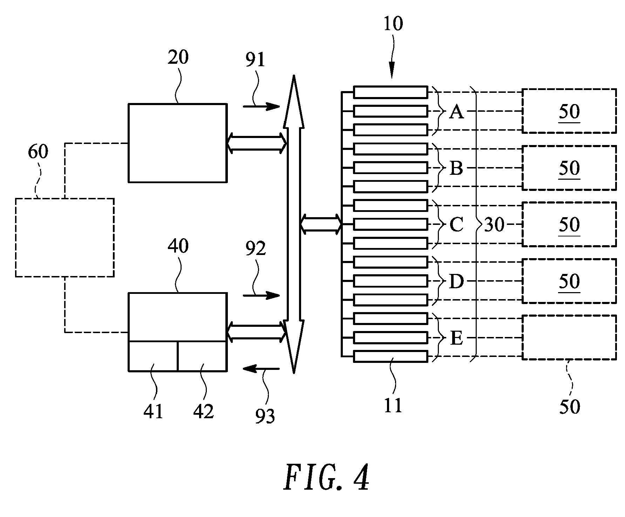

Referring to FIG. 4, the detection module 40 of the universal connection recognition system 100 may further include an analog signal generating unit 41 and an impedance detection unit 42. The analog signal generating unit 41 is configured to generate and send out an analog signal, and the impedance detection unit 42, to detect the impedance value between each two groups of detection pins and thereby obtain the impedance characteristics of the corresponding pin combination 30 and of the corresponding blood glucose meter (or other detection device).

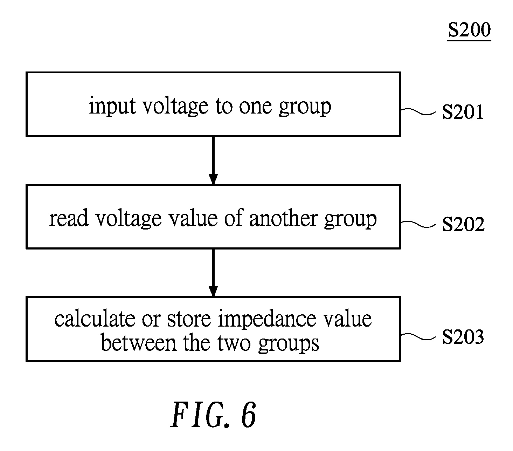

FIG. 6 shows an embodiment of the detection process S200 performed by the detection module 40. As stated above with regard to the function of the detection module 40, the detection process S200 may include: inputting a voltage to a group of detection pins (step S201), reading a voltage value from another group of detection pins (step S202), and calculating or storing an impedance value between the two groups of detection pins (step S203).

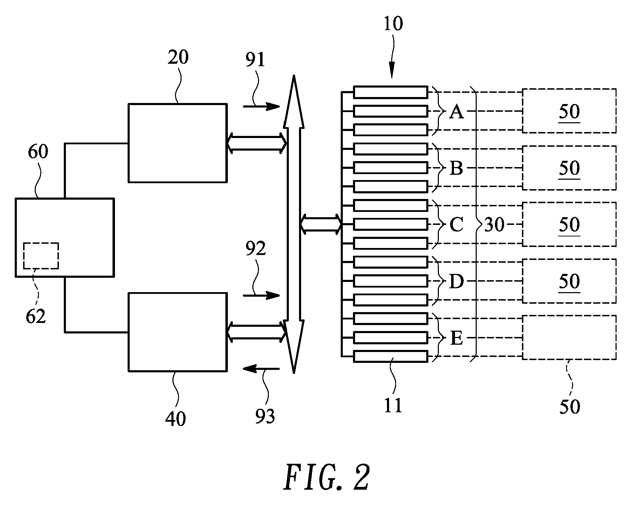

Moreover, referring to FIG. 2, the universal connection recognition system 100 may further include a control unit 60 for controlling the operation of the pin recognition module 20 and of the detection module 40. The control unit 60 can be a hardware device, software, firmware, or a combination of any two of the above.

The control unit 60 may further include or connect with a storage element for storing the data of blood glucose meters or other detection devices that have been connected to and detected by the system. When one of those blood glucose meters or detection devices is connected to the system again, the data corresponding to the blood glucose meter or detection device can be immediately retrieved from the storage element 62 and put to use.

In a nutshell, the universal connector 10, the pin recognition module 20, the detection module 40, and the control unit 60 are so designed that the universal connection recognition system 100 can be implemented at low cost without a complicated manufacturing process or costly production equipment, has a wide range of applications due to its compatibility with various detection instruments or devices, and features a learning function to ensure that the correct pin-grouping arrangement is automatically applied when a device is connected to the system again.

The embodiments described above are intended only to demonstrate the technical concept and features of the present invention so as to enable a person skilled in the art to understand and implement the contents disclosed herein. It is understood that the disclosed embodiments are not to limit the scope of the present invention.

Therefore, all equivalent changes or modifications based on the concept of the present invention should be encompassed by the appended claims.

## Claims

1. A universal connection recognition system (100), comprising:
a universal connector (10) composed of a plurality of detection pins (11);
a pin recognition module (20) for sending an identifying signal (91) to one of the detection pins (11), reading a signal from each of the other detection pins (11), and identifying and grouping the detection pins (11) to form a corresponding pin combination (30); and
a detection module (40) for sending a test signal (92) to the corresponding pin combination (30) and receiving and processing a feedback signal (93) sent by the corresponding pin combination (30) in response to the test signal (92),
wherein the detection pins (11) are electrically connected with a plurality of external terminals to be detected (50), and each of the external terminals to be detected (50) is electrically connected with at least one of the detection pins (11).

2. The universal connection recognition system (100) of claim 1, further comprising a control unit (60) for controlling the pin recognition module (20) and the detection module (40).

3. The universal connection recognition system (100) of claim 1 or 2, wherein the detection pins (11) are arranged in a two-dimensional or three-dimensional array.

4. The universal connection recognition system (100) of claim 1 or 2, wherein the pin recognition module (20) comprises a digital signal generating unit (21) and a pin recognition unit (22).

5. The universal connection recognition system (100) of claim 1 or 2, wherein the identifying signal (91) is a digital signal.

6. The universal connection recognition system (100) of claim 1 or 2, wherein the detection module (40) comprises an analog signal generating unit (41) and an impedance detection unit (42).

7. The universal connection recognition system (100) of claim 1 or 2, wherein the test signal (92) is an analog signal.

8. The universal connection recognition system (100) of claim 2, wherein the control unit (60) further comprises a storage element (62).

9. The universal connection recognition system (100) of claim 1 or 2, wherein the external terminals to be detected (50) are connection terminals of a blood glucose meter.

10. The universal connection recognition system (100) of claim 1, wherein each of the detection pins (11) is a gold finger or a pogo pin.

11. A pin recognition process (S100) for a plurality of detection pins (11), comprising the steps of:
looking for an unclassified one of the detection pin (11) (S 101 ), wherein pin-corresponding data are read to identify the unclassified one of the detection pins (11);
sending a signal to one of the detection pins (11) (S102), wherein an identifying signal (91) is sent to one of the detection pins (11);
detecting the other detection pins (11) (S103), wherein a signal is read from each of the other detection pins (11);
grouping the detection pins (11) (S104), wherein the detection pins (11) are identified and grouped according to the signal read from each of the other detection pins (11); and
establishing a corresponding pin combination (30) (S105), wherein the grouped detection pins (11) are defined as the corresponding pin combination (30)
